# EUROPEAN PATENT APPLICATION

(11) **EP 4 001 425 A1**
(43) Date of publication of application: **25.05.2022**
(21) Application number: 19938680.6
(22) Date of filing: 19.07.2019
(51) Int. Cl.: C12Q 1/06, C12N 5/071, C12N 5/0735, G01N 33/50, G01N 33/68

(54) **METHOD FOR EVALUATING STATE OF CELL DIFFERENTIATION**

(71) Applicant: Tokyo Electron Limited, Tokyo 107-6325 (JP); SHIMADZU CORPORATION, Kyoto-shi, Kyoto 604-8511 (JP); Foundation for Biomedical Research and Innovation at Kobe, Kobe-shi, Hyogo 650-0047 (JP)
(72) Inventor: HARA Keisuke, Tokyo 107-6325 (JP); GOMI Shinichi, Tokyo 107-6325 (JP); NAGATA Tomohisa, Sapporo-shi Hokkaido 060-0051 (JP); KAGAWA Kenichi, Tokyo 107-6325 (JP); OSHIMA Yasuhiro, Nirasaki-shi Yamanashi 407-0192 (JP); SUZUKI Takashi, Kyoto-shi Kyoto 604-8511 (JP); TAKAHASHI Masatoshi, Kyoto-shi Kyoto 604-8511 (JP); YAMAMOTO Takako, Kobe-shi Hyogo 650-0047 (JP); KAWAMATA Shin, Kobe-shi Hyogo 650-0047 (JP)
(74) Representative: Mewburn Ellis LLP
(86) International application number: PCT/JP2019/028556
(87) International publication number: WO 2021/014515

(57) **Abstract**

The present invention relates to a method for evaluating the state of cell differentiation during the process of inducing differentiation of human induced pluripotent stem cells (iPS cells) into retinal pigment epithelial cells. The present invention further relates to a method for evaluating the state of cell differentiation during the process of inducing differentiation of human embryonic stem cells (ES cells) into retinal pigment epithelial cells.

## Description

### Technical Field

The present invention relates to a method for evaluating the state of cell differentiation in the process of inducing differentiation of human induced pluripotent stem cells (iPS cells) into retinal pigment epithelial cells. The present invention further relates to a method for evaluating the state of cell differentiation in the process of inducing differentiation of human embryonic stem cells (ES cells) into retinal pigment epithelial cells.

### (Background Art)

Age-related macular degeneration is a disease in which the functionality of the macula in the center of the retina is degraded, and is one of the main causes of blindness among the elderly of developed countries. Age-related macular degeneration includes exudative and atrophic types. Retinal pigment epithelial (RPE) cells form a sheet-like monolayer of cells located outside the retina with macula and are important for maintaining photoreceptor cells in the retina. Choroid rich in blood vessels is further present outside the RPE cell layer. To obtain normal vision, the RPE cells and choroids on the outside of the retina need to function normally. Exudative age-related macular degeneration is a condition in which the function of photoreceptor cells is impaired due to subretinal bleeding or fluid leakage, from newly formed abnormal choroidal blood vessels under the retina through the retinal pigment epithelium as a result of functional deterioration or damage of RPE cells due to aging. Atrophic age-related macular degeneration is a disease of impaired visual acuity whereby RPE cell inflammation caused by aging results in the loss of both the overlying photoreceptor cells and the RPE cells.

As a treatment for exudative age-related macular degeneration, intraocular injection of a VEGF inhibitor such as an anti-vascular endothelial cell growth factor (VEGF) antibody is performed for the purpose of suppressing new blood vessels. However, since the therapeutic effect of anti-VEGF antibody has a short duration, there are many cases of recurrence. No effective treatment has been found for atrophic age-related macular degeneration. Since functional deterioration or damage of RPE cells are thought to cause both exudative and atrophic age-related macular degeneration, attempts have been made to induce differentiation of RPE cells from ES cells or iPS cells, which are pluripotent stem cells, for retinal transplant after removal of new blood vessels. For this purpose, a method for inducing differentiation of ES cells or iPS cells into RPE cells and a method for producing a sheet-like retinal pigment epithelium from the differentiated RPE cells have been developed (Patent Literature 1, Non-Patent Literature 1 and Non-Patent Literature 1, and Patent Literature 2).

In clinical studies in which human iPS cell-derived RPE cells were transplanted, the type of RPE cells that exhibit a high transplantation effect has not been fully elucidated. On the other hand, since the pigmentation (the amount of melanin) in RPE cells correlates well with the maturity of cells, a method for selecting RPE cells suitable for transplantation by observing pigmentation or measuring the amount of pigment has been proposed (Non-Patent Literature 3). However, since the measurement of pigmentation is possible after differentiation of iPS cells into RPE cells has progressed, and the RPE cells have matured, it is difficult to select the RPE cells with high suitability for transplantation before the pigment is deposited. Inducing differentiation of pluripotent stem cells into RPE cells usually requires long-term culture of several tens of days (Non-Patent Literatures 4 and 5). Therefore, predicting whether differentiated RPE cells will be obtained during culture is important in preparing for RPE cell transplantation. Non-Patent Literature 5 reports OTX1 and OTX2 as markers of progenitor cells that may differentiate from ES cells to RPE cells. However, in order to measure the expression level of OTX1 or OTX2, an invasive treatment to the cells, that is, crushing or lysing the cells, is required, making it impossible to evaluate the transplant cells.

### (Prior Art Literatures)

### (Patent Literature)

(Patent Literature 1) WO2014/030749

### (Non-patent Literatures)

(Non-patent Literature 1) Hirami Y, Osakada F, Takahashi K, et al. Generation of retinal cells from mouse and human induced pluripotent stem cells. Neurosci Lett. 2009;458:126-131.
(Non-patent Literature 2) Kamao H, Mandai M, Okamoto S, et al. Characterization of human induced pluripotent stem cell-derived retinal pigment epithelium cell sheets aiming for clinical application. Stem Cell Reports 2014;2:205-218.
(Non-patent Literature 3) Kamao H, Mandai M, Wakamiya S, et al. Objective evaluation of the degree of pigmentation in human induced pluripotent stem cell-derived RPE. Invest Ophthalmol Vis Sci. 2014;55:8309-8318.
(Non-patent Literature 4) Lane A, Philip LR, Ruban L, et al. Engineering efficient retinal pigment epithelium differentiation from human pluripotent stem cells. Stem Cells Transl Med. 2014;3:1295-1304.
(Non-patent Literature 5) Vugler A, Carr AJ, Lawrence J, et al. Elucidating the phenomenon of HESC-derived RPE: anatomy of cell genesis, expansion and retinal transplantation. Exp Neurol. 2008;214:347-361.

### (Outline of the Invention)

### (Problem to be Solved by the Invention)

In the process of differentiating human induced pluripotent stem cells (iPS cells) or human embryonic stem cells (ES cells) into retinal pigment epithelial cells (RPE cells), even under the same culture conditions, cells that differentiate into mature RPE cells and cells that remain undifferentiated are obtained. The objective of the present invention is to provide a method for early discrimination between cells that differentiate from pluripotent stem cells to RPE cells and cells that do not, by performing a non-invasive assessment of the cells before the pigmentation, which is an indicator of cell maturity, is observed.

### (Means for Solving the Problem)

That is, the object of the present invention is achieved by the following invention.
(1)
   A method for evaluating the differentiation state of pluripotent stem cells in the process of inducing differentiation pluripotent stem cells into retinal pigment epithelial cells, comprising
   (1) a step of measuring the amount of the indicator substance present in the culture supernatant of pluripotent stem cells, and
   (2) a step of evaluating the state of cell differentiation from pluripotent stem cells to retinal pigment epithelial cells based on changes in the amount of the indicator substance, where the indicator substances are ornithine and/or citrulline.
(2)
   The method according to (1), wherein in the step (2), the differentiation state of pluripotent stem cells is evaluated by analyzing the changes over time in the amount of the indicator substance present in the culture supernatant.
(3)
   The method according to (2), wherein the period for analyzing the changes over time is the period from day 0 to day 20, with the day when the medium for pluripotent stem cells is replaced from the medium for cell proliferation to the medium for cell differentiation set as day 0.
(4)
   The method according to (2), wherein the period for analyzing the changes over time is the period from day 3 to day 12, with the day when the medium for pluripotent stem cells is replaced from the medium for cell proliferation to the medium for cell differentiation set as day 0.
(5)
   The method according to any one of (2) to (4), wherein an assessment is made that cell differentiation are likely to progress from pluripotent stem cells to retinal pigment epithelial cells, when a significant changes over time is determined.
(6)
   The method according to (1), wherein in the step (1), the amount of ornithine and citrulline as the indicator substances is measured, and when the changes over time in the amount of ornithine and citrulline are substantially the same, an assessment is made that cell differentiation has progressed from pluripotent stem cells to retinal pigment epithelial cells.
(7)
   The method according to any one of (2) to (4), wherein in the step (2), the state of cell differentiation from pluripotent stem cells to retinal pigment epithelial cells is evaluated based on the threshold of the coefficient of variation regarding the amount of the indicator substance.
(8)
   The method according to (7), wherein when the coefficient of variation threshold is 0.20 or more for the amount of ornithine and/or 0.30 or more for the amount of citrulline, an assessment is made that cell differentiation from pluripotent stem cells to retinal pigment epithelial cells progresses.
(9)
   The method according to (1), further including step (3) of measuring the amount of the indicator substance present in the culture supernatant of the control cells whose state of cell differentiation is known, where
   the state of cell differentiation from pluripotent stem cells to retinal pigment epithelial cells is evaluated, by comparing the amount of the indicator substance present in the culture supernatant of the pluripotent stem cells measured in step (1), with the amount of the indicator substance present in the culture supernatant of the control cells measured or measured in step (3).
(10)
   The method according to (9), wherein the state of cell differentiation from pluripotent stem cells to retinal pigment epithelial cells is evaluated, based on whether the ratio or difference between the amount of the indicator substance present in the culture supernatant of the pluripotent stem cells and the amount of the indicator substance present in the culture supernatant of the control cells is equal to or greater than the predetermined threshold or less than the threshold.
(11)
   The method according to (9), wherein the state of cell differentiation from pluripotent stem cells to retinal pigment epithelial cells is evaluated, by comparing the changes over time of the amount of the indicator substance present in the culture supernatant of the pluripotent stem cells and the changes over time of the amount of the indicator substance present in the culture supernatant of the control cells.
(12)
   The method according to any one of (9) to (11), wherein the control cells are cells having the ability to remain undifferentiated in the step of inducing differentiation from pluripotent stem cells to retinal pigment epithelial cells.
(13)
   The method according to (1), wherein in the step of inducing differentiation of pluripotent stem cells into retinal pigment epithelial cells, the method further comprises step (4) of measuring in advance the amount of the indicator substance present in the culture supernatant of the control cells using cells in which cell differentiation has been confirmed as control cells,
   where the state of cell differentiation is evaluated by comparing the amount of the indicator substance present in the culture supernatant of pluripotent stem cells with the amount of the indicator substance present in the culture supernatant of the control cells.
(14)
   The method according to (13), wherein the state of cell differentiation is evaluated based on a threshold value determined based on the amount of the indicator substance present in the culture supernatant of the control cells.
(15)
   The method according to any one of (1) to (14), wherein the pluripotent stem cells are induced pluripotent stem cells (iPS cells) or embryonic stem cells (ES cells).
(16)
   The method according to any one of (1) to (15), wherein the amount of the indicator substance is measured by mass spectrometry.
(17)
   A method for producing retinal pigment epithelial cells from pluripotent stem cells using the method according to any one of (1) to (16).
(18)
   A method for evaluating the differentiation state of ES cells in the step of inducing differentiation of embryonic stem cells (ES cells) into retinal pigment epithelial cells, comprising
   (1) a step of measuring the amount of the indicator substance present in the culture supernatant of ES cells, and
   (2) a step of evaluating the state of cell differentiation from ES cells to retinal pigment epithelial cells based on the change in the amount of the indicator substance, where
      the indicator substance is at least one type selected from the group consisting of glutathione, ornithine, citrulline, cysteine, pipecolic acid, putrescine, proline, 2-aminoadipic acid, cytidine, deoxycytidine, adenosine, and inosine.
(19)
   A method for evaluating the differentiation state of ES cells in the step of inducing differentiation of embryonic stem cells (ES cells) into retinal pigment epithelial cells, comprising
   (1) a step of measuring the amount of the indicator substance present in the culture supernatant of ES cells, and
   (2) a step of evaluating the state of cell differentiation from ES cells to retinal pigment epithelial cells based on the change in the amount of the indicator substance, where
      the indicator substance is at least one type selected from the group consisting of glutathione, ornithine, citrulline, cysteine, pipecolic acid, 2-aminoadipic acid, cytidine, and deoxycytidine.
(20)
   A method for evaluating the differentiation state of ES cells in the step of inducing differentiation of embryonic stem cells (ES cells) into retinal pigment epithelial cells, comprising
   (1) a step of measuring the amount of the indicator substance present in the culture supernatant of ES cells, and
   (2) a step of evaluating the state of cell differentiation from ES cells to retinal pigment epithelial cells based on the change in the amount of the indicator substance, where
      the indicator substance is adenosine and/or inosine.
(21)
   The method according to any one of (18) to (20), wherein in step (2), the differentiation state of ES cells is evaluated by analyzing the changes over time in the amount of the indicator substance present in the culture supernatant.
(22)
   The method according to any one of (18) to (21), wherein the amount of the indicator substance is measured by mass spectrometry.
(23)
   A method for producing retinal pigment epithelial cells from ES cells using the method according to any one of (18) to (22).

### Effect of the Invention

According to the present invention, in the process of differentiating from induced pluripotent stem cells (iPS cells) or embryonic stem cells (ES cells), which are pluripotent stem cells, into retinal pigment epithelial cells (RPE cells), it is possible to distinguish at an early stage between cells that are differentiated into RPE cells and cells that are not, by measuring the indicator substance in the culture supernatant, before pigmentation (melanin), which is an indicator of differentiation into mature RPE cells, occurs. Long-term culture is required to induce differentiation from pluripotent stem cells to RPE cells, but being able to determine the success or failure of differentiation at an early stage can exclude early the cells that cannot be used for transplantation because they cannot be differentiated. This leads to saving labor and time that otherwise occurred from unnecessary culture costs and culture operation. Moreover, the methods of the invention are non-invasive to cells, since the indicator substances are measured in cell supernatants, so there is no need for invasive treatment of the cells, such as crushing or lysing the cells; the success or failure of inducing differentiation of the cells themselves that are actually planned to be transplanted can be evaluated in advance. Therefore, preparations for transplantation of RPE cells can be smoothly performed.

### Brief Description of the Drawings

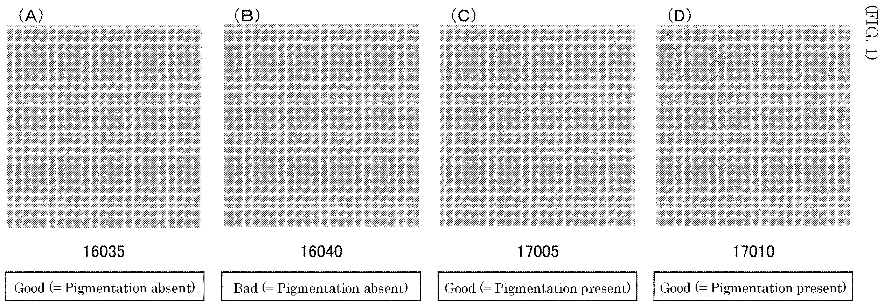

(FIG. 1) is a drawing showing culture of human iPS cells in independent culture vessels A (16035), B (16040), C (17005), and D (17010) for 75 days (A), 76 days (B), 99 days (C), and 91 days (D), respectively, and photographed by the reflection type bright-field observation apparatus. Pigmentation was observed in A, C and D (indicated as "Good (= Pigmentation present)"), and differentiation into retinal pigment epithelial cells was observed (results not shown). No pigmentation was observed in B (indicated as "Bad (= Pigmentation absent )"), and differentiation into retinal pigment epithelial cells was not observed.

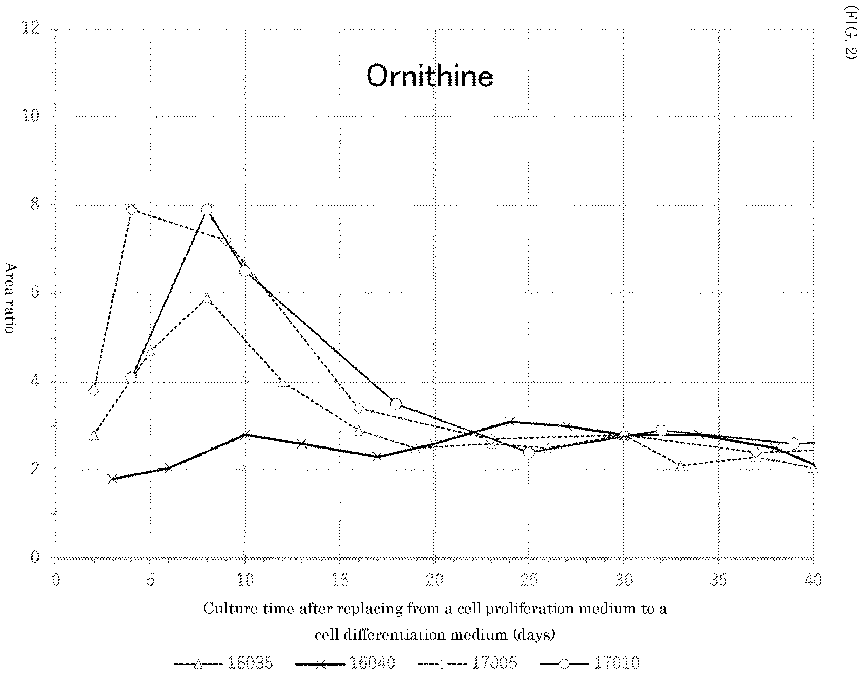

(FIG. 2) shows the results of culturing human iPS cells in independent culture vessels (16035, 16040, 17005, and 17010) and measuring the amount of ornithine in each culture vessel. The culture vessels 16035, 16040, 17005, and 17010 are the same as the culture vessels shown in FIG. 1. In the iPS cells of culture vessels 16035, 17005, and 17010 where pigmentation was finally observed, the day when the medium for cell proliferation was replaced with the medium for cell differentiation was set as day 0; a significant increase in the amount of ornithine was noted during the period from day 3 to day 12, but no pigmentation was observed during that period. In 16040, no significant increase in the amount of ornithine was observed.

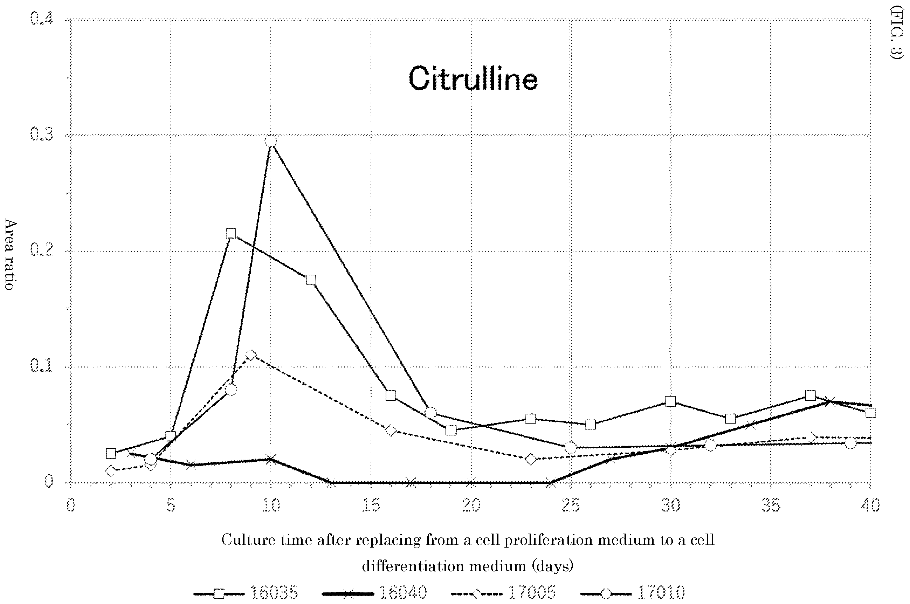

(FIG. 3) shows the results of culturing human iPS cells in independent culture vessels (16035, 16040, 17005, and 17010) and measuring the amount of citrulline in each culture vessel. The culture vessels 16035, 16040, 17005, and 17010 are the same as the culture vessels shown in FIG. 1. In the iPS cells of culture vessels 16035, 17005, and 17010 where pigmentation was finally observed, the day when the medium for cell proliferation was replaced with the medium for cell differentiation was set as day 0; a significant increase in the amount of citrulline was noted during the period from day 5 to day 12, but no pigmentation was observed during that period. In 16040, no significant increase in the amount of citrulline was observed.

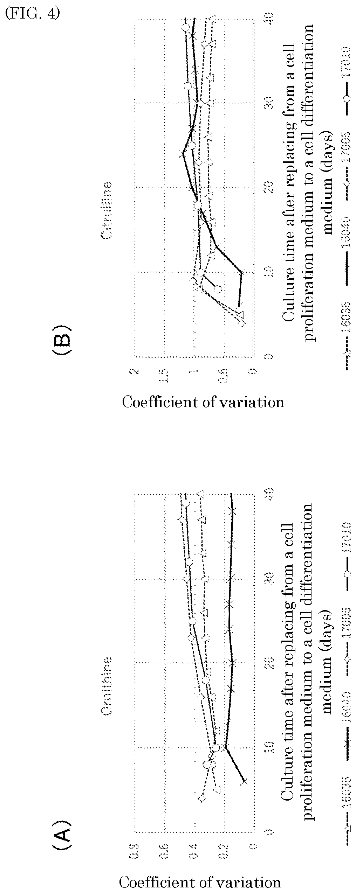

(FIG. 4) is a drawing showing changes over time in the amounts of ornithine (A) and citrulline (B) produced by human iPS cells as coefficients of variation. iPS cells were cultured in independent culture vessels (16035, 16040, 17005, and 17010) and the amount of ornithine (A) and citrulline (B) in each culture vessel was measured. The culture vessels 16035, 16040, 17005, and 17010 are the same as the culture vessels shown in FIG. 1. The number of data is the number of measurements of the amount of ornithine or citrulline during the cell culture period. The coefficient of variation was calculated based on the mean value and standard deviation of the amount measured each time.

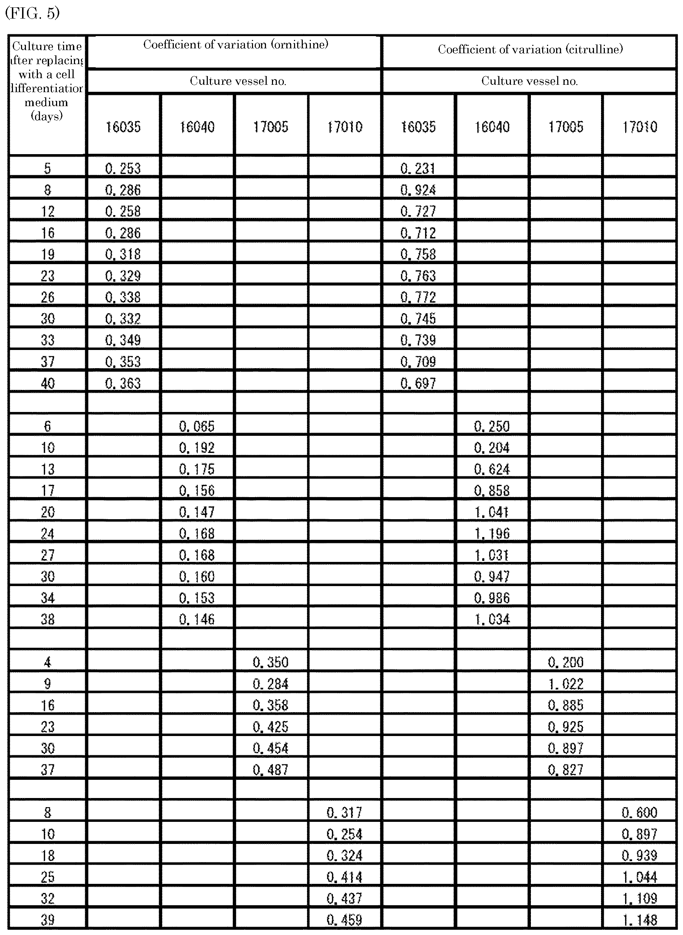

(FIG. 5) is a drawing showing the coefficient of variation of the amount of ornithine and citrulline shown in FIG. 4 numerically for each culture vessel.

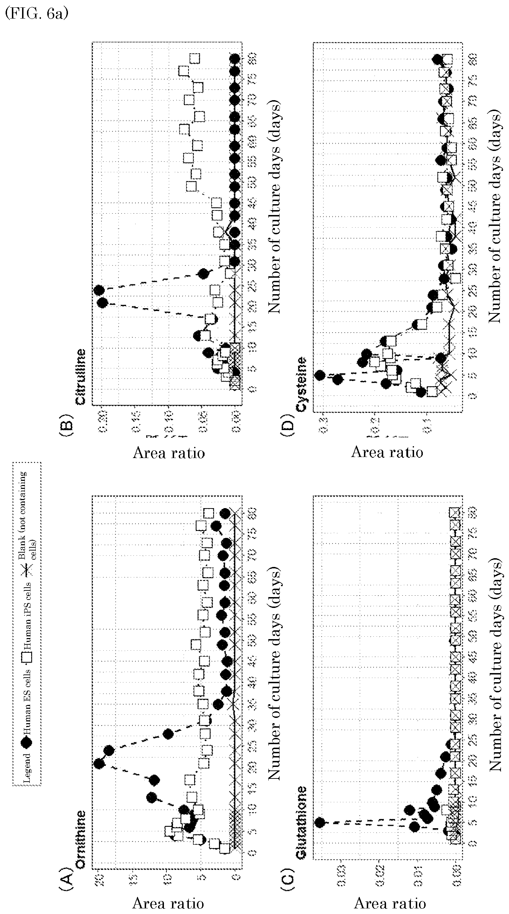

(FIG. 6a) is a drawing showing the results of culturing human ES cells and human iPS cells and measuring the amount of indicator substances present in the culture supernatant. It is the measurement result of (A) ornithine, (B) citrulline, (C) glutathione and (D) cysteine. The human ES cells used were observed to differentiate into retinal pigment epithelial cells, but human iPS cells did not. In the case of only the cell-free medium (blank), almost no changes over time in the amount of the indicator substance present in the culture supernatant with time was observed.
(FIG. 6b) is a drawing showing the results of culturing human ES cells and human iPS cells and measuring the amount of the indicator substance present in the culture supernatant. It is the measurement result of (E) pipecolic acid, (F) putrescine, (G) proline and (H) 2-aminoadipic acid. The human ES cells used were observed to differentiate into retinal pigment epithelial cells, but human iPS cells did not. In the case of only the cell-free medium (blank), almost no changes over time in the amount of the indicator substance present in the culture supernatant with time was observed.
(FIG. 6c) is a drawing showing the results of culturing human ES cells and human iPS cells and measuring the amount of the indicator substance present in the culture supernatant. It is the measurement result of (I) cytidine, (J) deoxycytidine, (K) adenosine and (L) inosine. The human ES cells used were observed to differentiate into retinal pigment epithelial cells, but human iPS cells did not. In the case of only the cell-free medium (blank), almost no changes over time in the amount of the indicator substance present in the culture supernatant with time was observed.

### Best Mode for Carrying Out the Invention

The present invention provides a method for evaluating the differentiation state of pluripotent stem cells in the process of inducing differentiation of pluripotent stem cells into retinal pigment epithelial cells. The method comprises a process of assessing the state of cell differentiation from pluripotent stem cells to retinal pigment epithelial cells, based on (1) the step of measuring the amount of the indicator substance present in the culture supernatant of pluripotent stem cells, and (2) the amount of the indicator substance. The indicator substances are ornithine and/or citrulline.

The present invention further provides a method for evaluating the differentiation state of ES cells in the process of inducing differentiation of embryonic stem cells (ES cells) into retinal pigment epithelial cells. The method comprises a process of assessing the state of cell differentiation from ES cells to retinal pigment epithelial cells, based on (1) the step of measuring the amount of the indicator substance present in the culture supernatant of ES cells, and (2) the changes in the amount of the indicator substance. The indicator substance is at least one type selected from the group consisting of glutathione, cysteine, deoxycytidine, 2-aminoadipic acid, citrulline, pipecolic acid, putrescine, ornithine, cytidine, adenosine, inosine, and proline. The indicator substance may be at least one type selected from the group consisting of glutathione, deoxycytidine, 2-aminoadipic acid, citrulline, pipecolic acid, ornithine and cytidine.

In the step of inducing differentiation in the present invention, retinal pigment epithelial cells are produced from pluripotent stem cells. iPS cells (Induced Pluripotent Stem cells) or ES cells (Embryonic Stem cells), which are undifferentiated cells, can be used as pluripotent stem cells. iPS cells are artificial stem cells and can be produced by introducing specific pluripotent inducers such as nucleic acids, proteins, or low-molecular-weight compounds into somatic cells (Takahashi K., Yamanaka D., Cell, 2006; 126: 663-676, Takahashi K. et al. Cell, 2007; 131: 861-872, WO2007/069666). ES cells can be produced by removing the inner cell mass from the blastocyst of a fertilized mammalian egg and culturing this inner cell mass (Suemori H. et al. Biochem Biophys Res Commun. 2006; 345: 926-932). As iPS cells and ES cells, cells derived from mammals such as humans, monkeys, mice, rats, dogs, cats, cows, horses, pigs, sheep, goats, rabbits, hamsters, guinea pigs, etc. can be used; human-derived cells are preferable.

In the process of inducing differentiation of iPS cells or ES cells, which are pluripotent stem cells, into retinal pigment epithelial cells, the stem cells are first cultured in a cell proliferation medium and then replaced with a cell differentiation medium for cultivation. As the culture conditions and media for differentiating the stem cells into retinal pigment epithelial cells, culture conditions and media known to those skilled in the art can be used, but can also be appropriately modified by those skilled in the art. A commercially available medium may be used, and an example of the cell proliferation medium includes Essential 8 (registered trademark) medium, and an example of the cell differentiation medium includes Essential 6 (registered trademark) medium.

Several methods have been reported as methods for confirming the differentiation of iPS cells or ES cells into retinal pigment epithelial cells (Ministry of Health, Labor and Welfare, The Evaluation and Licensing Division, Pharmaceutical and Food Safety Bureau, PFSB/ELD/OMDE Notification No. 0529, No. 1 (May 29, 2013) Attachment 1 "Evaluation criteria for retinal pigment epithelial cells derived from autologous iPS cells"). For example, the differentiation into retinal pigment epithelial cells can be confirmed by observing the presence of brown pigment (melanin) deposition unique to retinal pigment epithelial cells or polygonal or paving stone-like cell morphology by observation using a phase-contrast microscope, or the like. In addition, by confirming the expression of characteristic genes in retinal pigment epithelial cells, differentiation into retinal pigment epithelial cells can be confirmed. Characteristic genes of retinal pigment epithelial cells include RPE65, CRALBP, MERTK, and BEST1. The mixture of iPS cells or ES cells (undifferentiated cells) can be evaluated, by immunostaining analysis of undifferentiated markers such as Oct3/4, Sox, and TRA-1-60, or by measurement of undifferentiated marker genes (OCT3/4, Nanog, and Lin28, etc.)

With the method of the present invention, it is possible to identify the differentiated cells or cell population at an early stage before the differentiation of iPS cells or ES cells, which are pluripotent stem cells, into retinal pigment epithelial cells is confirmed. For that purpose, in process (1) of the present invention, the amount of the indicator substance present in the culture supernatant of iPS cells or ES cells is measured. The indicator substance is a compound released into the culture supernatant as a metabolite in iPS cells or ES cells, or a medium component contained in a culture medium for differentiation, and ornithine (ornithine) and citrulline (citrulline), which are a kind of amino acids, are preferable. For ES cells, in addition to ornithine and citrulline, as index substances for early discrimination of cells or cell populations that differentiate from ES cells to retinal pigment epithelial cells, glutathione, cysteine, pipecolic acid, putrescine, proline, 2-aminoadipic acid, cytidine, deoxycytidine, adenosine, and inosine can be selected. These can be used alone or in combination of two or more to evaluate the state of cell differentiation.

The process (2) of the present invention is a process of evaluating the state of cell differentiation from the pluripotent stem cell to retinal pigment epithelial cells based on the amount of the indicator substance present in the culture supernatant of the pluripotent stem cells measured in process (1) of the present invention. In one embodiment of the present invention, the differentiation state of pluripotent stem cells is evaluated by analyzing the changes over time in the amount of the indicator substance. The changes over time in the amount of the indicator substance is a change in the amount of the indicator substance present in the culture supernatant as the time of culturing the pluripotent stem cells passes. The amount of the indicator substance present in the culture supernatant can be indicated by the concentration or absolute amount of the indicator substance present in the culture solution, the amount of the indicator substance per unit cell number, the total amount of the indicator substance per culture vessel, and the like; but it is not limited thereto. Analyzing the changes over time in the amount of the indicator substance present in the culture supernatant includes detecting transient or persistent increases or decreases in the amount in graphs or profiles of changes over time in the amount of the indicator substance. A transient or persistent increase or decrease in the amount of the indicator substance present in the culture supernatant is recognized as a variation from the baseline of the graph or profile of the amount of existence over time.

In one embodiment of the invention, the period for analyzing changes over time in the amount of ornithine and/or citrulline present in the culture supernatant is preferably the period from day 0 to day 20, where the day when the medium for pluripotent stem cells was replaced from q cell proliferation medium to a cell differentiation medium is set as day 0. The analysis of the changes over time may be performed over the entire period, but as long as the change is observed, it may be analyzed in a shorter period, for example, from day 3 to day 12. The amount of ornithine may be analyzed in the period from day 4 to day 12, and the amount of citrulline may be analyzed in the period from day 8 to day 12. Usually, the replacement of the cell proliferation medium with the cell differentiation medium is preferably carried out after culturing in the cell proliferation medium for 7 to 10 days, but the number of days may be increased or decreased depending on the degree of cell proliferation.

In one embodiment of the present invention, when it is determined that the amount of ornithine or citrulline present in the culture supernatant of pluripotent stem cells changes significantly with time, an assessment is made that cell differentiation from the pluripotent stem cells to retinal pigment epithelial cells is likely to progress. A determination of a significant changes over time refers to large fluctuations or divergence from the baseline of the graph or profile of the changes in the amount of ornithine or citrulline over time, rapidly increasing and then decreasing the amount of ornithine or citrulline. As a result, a clear peak is observed in the changes over time in the amount of ornithine or citrulline. Those skilled in the art can easily recognize the peak by referring to the graph or profile of the changes over time. The peak of the changes over time may be a unimodal peak or a multimodal peak.

In order for iPS cells or ES cells, which are pluripotent stem cells, to differentiate into retinal pigment epithelial cells and pigmentation, which is an indicator of differentiation, to occur, a culture time of about 30 to 50 days is usually required. On the other hand, the peak in the changes over time in the amount of ornithine or citrulline present in the culture supernatant is observed before the time when pigmentation occurs in the cells. Since the cultured cells whose peak was observed has pigmentation, which is the index of differentiation into retinal pigment epithelial cells, occurs by continuing the culture thereafter, the moment the peak was observed, an assessment is made that cell differentiation from pluripotent stem cells to retinal pigment epithelial cells is likely to progress.

In one embodiment of the present invention, when the changes over time in the amounts of ornithine and citrulline present in the culture supernatant of pluripotent stem cells are substantially identical, an assessment is made that cell differentiation from the pluripotent stem cells to retinal pigment epithelial cells progresses. Since the evaluation is based on both the changes in the amounts of ornithine and citrulline over time, the reliability of the evaluation results is higher than when the evaluation is performed using the change in the amount of either ornithine or citrulline alone. Substantially the same changes over time in the amounts of ornithine and citrulline present in the culture supernatant refers to the changes over time that satisfies the following two conditions. The conditions are (1) it is common that the peak observed in the amounts of ornithine and citrulline changes over time is a peak having a form of amount increasing as the culture time progresses and then decreasing; (2) even if the peak is multimodal, the culture time for obtaining the main peak with the highest amount is almost the same. The term 'almost the same' refers to the difference in culture time giving the main peak position between ornithine and citrulline is within 5 days, preferably within 3 days, and more preferably within 1 day.

In one embodiment of the invention, the state of cell differentiation from pluripotent stem cells to retinal pigment epithelial cells is assessed based on the coefficient of variation threshold for the amount of ornithine and/or citrulline, both of which are the indicator substances. The coefficient of variation is calculated by dividing the standard deviation of the amount by the mean value of the amount. That is, during the culture period of pluripotent stem cells, the number of times the amount of the indicator substance is measured is used as the number of data, and the mean value of the amount measured each time is calculated. Next, the standard deviation is calculated using the positive square root of the value obtained by dividing the sum of the squares of the difference between the measured amount and the mean value of the amount at each time by the number of data. The coefficient of variation is calculated by dividing the standard deviation calculated in this way by the mean value of the amount. The coefficient of variation is the ratio of the magnitude of the variation of the data, showing that the larger the coefficient of variation is, the larger the variation in the amount of the indicator substance measured during the culture period is, and conversely, the smaller the coefficient of variation is, the smaller the variation in the amount of the indicator substance measured during the culture period is.

In one embodiment of the invention, when the threshold of the coefficient of variation is 0.20 or more of the amount of ornithine, preferably 0.25 or more and/or 0.30 or more of the amount of citrulline, an assessment is made that cell differentiation from pluripotent stem cells to retinal pigment epithelial cells progresses. That is, when the amount of ornithine and/or citrulline present in the culture supernatant shows a certain fluctuation during the culture period, an assessment is made that the cell differentiation progresses. The coefficient of variation threshold for the amount of ornithine and/or citrulline is a value empirically derived from the comparison between the calculated value of the coefficient of variation and the state of cell differentiation from pluripotent stem cells to retinal pigment epithelial cells. Those skilled in the art can also reset the more appropriate thresholds estimated according to the test examples or the number of tests to be compared. The culture period for calculating the coefficient of variation can be arbitrarily set as long as it is after the day when the medium for pluripotent stem cells is replaced with the medium for cell proliferation to the medium for cell differentiation. The culture period is preferably the period from day 0 to day 20, more preferably day 3 to day 12, where the day when the medium for pluripotent stem cells was replaced with the medium for cell proliferation to the medium for cell differentiation was set as day 0. For the amount of ornithine, the culture period is more preferably from day 4 to day 12, and for the amount of citrulline, the period from day 8 to day 12 is more preferable.

One embodiment of the invention further comprises process (3) of measuring the amount of the indicator substance (ornithine and/or citrulline) present in the culture supernatant of the control cells whose differentiation state has already been known, and provides a method for assessing the state of cell differentiation from pluripotent stem cells to retinal pigment epithelial cells, by comparing the amount of the indicator substance present in the culture supernatant of the pluripotent stem cells measured in process (1) of the present invention with the amount of the indicator substance present in the culture supernatant of the control cells to be measured or has been measured in process (3). The process (3) for measuring the amount of the indicator substance present in the culture supernatant of the control cell may be performed at the same time as process (1), or may be performed independently of process (1), or may be carried out before performing process (1). The control cells may be pluripotent stem cells derived from the same cell line as the cell subjected to the induction of differentiation from the pluripotent stem cells to the retinal pigment epithelial cells, or they may be cells derived from pluripotent stem cell line different from the cell subjected to differentiation induction. Control cells in which the state of cell differentiation is known are cells whose state of differentiation is known, whether they differentiated or not, and the differentiation state does not change during the culture period. The undifferentiated state can be confirmed by immunostaining, for example, the presence of undifferentiated markers such as Oct3/4, Sox, and TRA-1-60, or by measurement of undifferentiated marker genes (OCT3/4, Nanog, Lin28, etc.). The differentiated state can be confirmed, for example, in the case of retinal pigment epithelial cells, by the presence of brown pigment (melanin) deposition or polygonal or paving stone-like cell morphology, or by measuring gene expression such as RPE65, CRALBP, MERTK and BEST1.

One embodiment of the present invention provides a method for evaluating the state of cell differentiation from pluripotent stem cells to retinal pigment epithelial cells based on whether the ratio or difference of the amount of indicator substances (ornithine and/or citrulline) present in the culture supernatant of the pluripotent stem cells and the amount of the indicator substance present in the culture supernatant of the control cells is equal to or greater than a predetermined threshold or less than the threshold. Regarding the culture supernatants of cells in which differentiation from pluripotent stem cells to retinal pigment epithelial cells was confirmed and cells in which differentiation was not confirmed, the threshold can be derived by increasing the number of cell culture samples and repeatedly measuring the amount of ornithine and citrulline present in each sample. That is, the threshold value is an empirically derived value and can be easily set by those skilled in the art.

One embodiment of the present invention provides a method for evaluating the state of differentiation of pluripotent stem cells into retinal pigment epithelial cells, by comparing the changes over time in the amount of the indicator substance (ornithine and/or citrulline) present in the culture supernatant of the pluripotent stem cells with the changes over time in the amount of the indicator substance present in the culture supernatant of the control cells. The changes over time in the amount of the indicator substance are changes in the amount of the indicator substances present in the culture supernatant as the time of culturing both pluripotent stem cells and control cells passes. The amount of the indicator substance can be indicated by the concentration or absolute amount of the indicator substance present in the culture solution, the amount of the indicator substance per unit cell number, the total amount of the indicator substance per culture vessel, and the like; but it is not limited thereto. Comparing the changes over time in the amount of the indicator substance present in the culture supernatant is to grasp the similarity or dissimilarity of the graph or profile of the changes in the amount of the indicator substance over time.

In one embodiment of the present invention, as the control cells, cells remained undifferentiated may be used in the process of inducing differentiation from pluripotent stem cells to retinal pigment epithelial cells. Cells remaining undifferentiated in the process of inducing differentiation from pluripotent stem cells to retinal pigment epithelial cells refers to cells that do not differentiate from pluripotent stem cells to retinal pigment epithelial cells, indicating that no pigmentation (melanin) is observed, or the expression of characteristic genes of retinal pigment epithelial cells such as RPE65, CRALBP, MERTK, and BEST1 remains unobserved. Keeping pluripotent stem cells in their undifferentiated state is achieved by subculturing them in the cell proliferation medium without replacing the medium for pluripotent stem cells with a cell differentiation medium.

The process of inducing differentiation from pluripotent stem cells to retinal pigment epithelial cells in one embodiment of the invention uses cells with confirmed cell differentiation as the control cells and further comprises process (4) of preliminarily measuring the amount of the indicator substance (ornithine and/or citrulline) present in the culture supernatant of the control cells, where a method for evaluating the state of cell differentiation is provided by comparing the amount of the indicator substance present in the culture supernatant of the pluripotent stem cells with the amount of the indicator substance present in the culture supernatant of the control cells. In this method, the amount of the indicator substance present in the culture supernatant is measured in advance for control cells in which differentiation from pluripotent stem cells to retinal pigment epithelial cells has been confirmed, and the measured value is used as a reference value or an index for determining that inducing differentiation has been performed. According to this method, based on the reference value or indicator, it is possible to determine whether the cultured pluripotent stem cells differentiate into retinal pigment epithelial cells. For the comparison between the amount of the indicator substance present in the culture supernatant of the pluripotent stem cells and the amount of the indicator substance present in the culture supernatant of the control cells, a comparison can be made on the amount of the substance present at a specific culture time. In one embodiment of the present invention, the state of differentiation of pluripotent stem cells into retinal pigment epithelial cells can be evaluated based on a threshold determined based on the amount of the indicator substance present in the culture supernatant of the control cells.

In one embodiment of the present invention, information on the differentiation state of a plurality of types of control cells and the amount of indicator substance (ornithine and/or citrulline) present in the culture supernatant of the control cells obtained in process (4) can be recorded as a library for each control cell. By using this library, it is also possible to evaluate the state of cell differentiation by collating the amount of the indicator substance present in the culture supernatant of the pluripotent stem cells in the process of inducing differentiation with the information on the amount of the substance present in the library corresponding to the same cell line as the pluripotent stem cells in the process of inducing differentiation.

Comparing the amount of the indicator substance present in the culture supernatant of the pluripotent stem cells and the amount of the indicator substance present in the culture supernatant of the control cells also includes comparing the amounts of the substance present at several culture times, that is, comparing graphs or profiles of changes in the amount over time. This is because comparing the amounts of the substance present at several culture times improves accuracy of determining whether the cells have differentiated or not. Whether they are differentiated or not can be determined based on the similarity of the graphs or profiles. Here, the similarity means that the shape of the graph or profile is similar as the culture time progresses.

In the present invention, as a method for quantitatively measuring the amount of the indicator substance present in the culture supernatant, a gas chromatograph method (GC), a liquid chromatograph method (LC), a mass spectrometry method (MS), or the like, can be used. Further, liquid chromatograph mass spectrometry (LC-MS) and gas chromatograph mass spectrometry (GC-MS) can be suitably used for quantitative measurement of indicator substances, but it is not limited thereto.

The analysis by LC-MS can be performed as follows. To 100 µL of the culture supernatant sample, 20 µL of 0.5 mM isopropylmalic acid aqueous solution is added as an internal standard, and after it is mixed, and 200 µL of acetonitrile or methanol is added for deproteinization. The obtained sample is centrifuged at 15,000 rpm for 15 minutes at room temperature using a Tomy Seiko centrifuge, and then the supernatant is recovered, diluted 10-fold with ultrapure water (MiLLi-Q (registered trademark) water, Merck Co., Ltd.) and subjected to LC-MS analysis. LC-MS analysis is carried out according to the analysis conditions recorded in "LC/MS/Method Package for Cell Culture Profiling" (hereinafter abbreviated as "MP") product of Shimadzu Corporation. MP is a collection of analytical condition parameters for analyzing compounds contained in a medium and metabolites secreted from cells by LC-MS. A compound is identified by the following criteria: the difference between the retention time of the standard product registered in MP and the retention time of the compound in the sample is within ± 0.3 minutes, both peaks of quantitative ions and confirmation ions are detected, and the strength value is 1000 or more. The compound is quantified by a method of calculating the area of the mass chromatogram for the characteristic ions (quantitative ions) of each compound in the sample.

The analysis by GC-MS can be performed as follows. To 100 µL of the culture supernatant sample, 10 µL of 0.5 mg/mL isopropylmalic acid aqueous solution is added as an internal standard, and after it is mixed, and 200 µL of acetonitrile is added for deproteinization. The obtained sample is centrifuged at 15,000 rpm for 15 minutes at room temperature, and then 100 µL of the supernatant is collected and dried under reduced pressure. Each sample is incubated in a pyridine solution containing methoxyamine hydrochloride to methoximate the compounds in the sample Additionally, MSTFA (N-methyl-N-trimethylsilyltrifluoroacetamide) is added to each sample to trimethylsilylate (derivatize) the compounds in the sample. The samples are subjected to a GC-MS analysis, after these derivatization treatments.

In GC-MS analysis, "Smart Metabolites Database" (hereinafter abbreviated as "DB") product of Shimadzu Corporation is used. The DB is a collection of data obtained by conducting GC-MS analyses on the standard products of various compounds subjected to derivatization treatments similar to the previously described one. The criteria used for the identification of the compounds are as follows: whether the difference between the retention index (a numerical value showing a relative retention time) specified in the DB and the retention index of a derivatized compound in the sample was within ±5, and whether both the quantitative ions and confirmation ions designated in the DB are detected or not for the derivatized compound in the sample. The quantification of compounds is performed by a method of calculating the area of a mass chromatogram created for characteristic ions of each derivatized compound in the sample according to the conditions specified in the DB.

When, for example, GC, LC, GC-MS, or LC-MS is used as the amount of the indicator substance present in the culture supernatant, the peak area value (Area) of the indicator substance in these measurement methods, or the concentration (Concentration) of the indicator substance in the culture supernatant calculated from the peak area value (Area) of the indicator substance can be used. Furthermore, "Area/Confluency" or "Concentration/Confluency" corrected (normalized) by the density of cells (Confluency), or "Area/Cell Number" or "Concentration/Cell Number" corrected by the number of cells (Cell Number or Cell Count) may also be used. By using the value corrected (normalized) by the cell density (Confluency) or the number of cells (Cell Number or Cell Count), even if there are differences in cell proliferation rates between the pluripotent stem cells and the control cells in the process of inducing differentiation, it is possible to compare the peak area value (Area/Confluency) of the indicator substance or the concentration (Concentration/Confluency) of the indicator substance per number of cells among the cells. Therefore, it is possible to evaluate the state of cell differentiation with high accuracy.

The present invention provides a method for producing retinal pigment epithelial cells from pluripotent stem cells, comprising a method for evaluating the differentiation state of the pluripotent stem cells of the present invention. Pluripotent stem cells include iPS cells and ES cells. With the method for producing retinal pigment epithelial cells of the present invention, the cells that differentiate into retinal pigment epithelial cells and cells that do not differentiate can be determined in the process of producing retinal pigment epithelial cells from pluripotent stem cells. The method for producing retinal pigment epithelial cells of the present invention can include a process of removing cells or cell populations that are determined not to differentiate into retinal pigment epithelial cells.

### Examples of Embodiment

Next, the present invention will be described in detail with reference to examples, but the scope of the present invention is not limited thereto.

### Example 1

### [Differentiation of human iPS cells into retinal pigment epithelial cells]

Human iPS cells were seeded in a tissue culture flask coated with vitronectin and cultured at 5% CO₂, 37°C. using Essential 8 (Invitrogen, registered trademark) as a medium for cell proliferation. On the 7th day after culturing, the cell proliferation medium was replaced with a cell differentiation medium (TeSR-E6, STEMCELL Technologies), and the cells were further cultured under 5% CO₂ at 37°C. The cells were observed with a reflective bright-field observation device 75 to 99 days after culturing. The pigmentation of cells in culture vessels 16035 (FIG. 1A), 17005 (FIG. 1C), and 17010 (FIG. ID) were observed, indicating successful cell differentiation into retinal pigment epithelial cells. However, despite culturing under the same conditions, no pigmentation was observed in the culture vessel 16040 (FIG. 1B), indicating unsuccessful cell differentiation into retinal pigment epithelial cells.

### Example 2

### [Measurement of ornithine and citrulline in culture supernatant of human iPS cells]

The amounts of ornithine and citrulline in the culture supernatant of the human iPS cells cultured in Example 1 were measured over time by LC-MS (FIGS. 2 and 3 respectively). The analyzer used was LCMS-8050 (Shimadzu Corporation). In the culture supernatants of the cells of the culture vessels 16035, 17005, and 17010 in which pigmentation was observed, and differentiation into retinal pigment epithelial cells was observed, the day when the cell proliferation medium was replaced with the cell differentiation medium was set as day 0, where a unimodal peak in ornithine amount was observed during the period from day 3 to day 12. In addition, a unimodal peak in citrulline amount was observed during the period from day 5 to day 15, with the day when the medium for cell proliferation was replaced with the medium for cell differentiation was set as day 0. On the other hand, in the culture supernatant of the cells in the culture vessel 16040, in which no pigmentation was observed, and the cells remained undifferentiated, the amount of ornithine did not fluctuate much, and no clear peak was observed in the change in the amount of ornithine over time until day 40, using the day when the medium for cell proliferation was replaced with the medium for cell differentiation as day 0. The amount of citrulline in the culture supernatant of cells in the culture vessel 16040 tended to gradually increase from around day 25, with the day when the cell proliferation medium was replaced with the cell differentiation medium as day 0, but no clear peak was observed.

In the cells of culture vessels 16035, 17005, and 17010, pigmentation was observed around day 40, with the day when the cell proliferation medium was replaced with the cell differentiation medium as day 0. In contrast, peaks of the amounts of ornithine and citrulline were observed earlier, in the period from day 3 to day 12 and the period from day 5 to day 15, respectively. This result shows that whether cells differentiate or not can be determined, before iPS cell differentiation into retinal pigment epithelial cells was confirmed by pigmentation, by measuring and analyzing the amount of ornithine and/or citrulline present in the culture supernatant. Conversely, it shows that differentiation is unlikely in cells that do not have peaks of the amount of ornithine and citrulline, even if the culture is continued. These show that cell differentiation can be predicted by analyzing the changes over time in the amount of ornithine and/or citrulline present in the culture supernatant.

Changes over time in the amount of ornithine and citrulline present in the culture supernatant of human iPS cells were displayed as the coefficient of variation. The results are shown in FIGS. 4A and 4B, respectively. In addition, a list of numerical values of the coefficient of variation is shown in FIG. 5. The culture time (days) shown in FIGS. 4A, 4B, and 5 is shown with the day when the medium for cell proliferation was replaced with the medium for cell differentiation used as day 0. The coefficient of variation of the amount of ornithine present in the culture supernatants of cells in culture vessels 16035, 17005, and 17010 differentiated into retinal pigment epithelial cells was given a value of 0.20 or more, at least in the period from day 4 to day 12 within the period from day 0 to day 20, which peaks in the change in the amount over time. On the other hand, in the culture supernatant of the cells in the culture vessel 16040, which was shown to remain undifferentiated, the coefficient of variation was less than 0.20 during the period from day 6 to day 20. The coefficient of variation of the amount of citrulline present in the culture supernatants of cells in culture vessels 16035, 17005, and 17010 differentiated into retinal pigment epithelial cells was given a value of 0.60 or more, at least in the period from day 8 to day 12 within the period from day 0 to day 20, which peaks in the change in the citrulline amount over time. On the other hand, in the culture supernatant of the cells in the culture vessel 16040, which was shown to remain undifferentiated, the coefficient of variation was less than 0.30 during the period from day 8 to day 12.

### Example 3

### [Comparison of indicator substances in culture supernatants of human ES cells and human iPS cells]

Human ES cells and human iPS cells were seeded on a 6-well plate (FALCON) coated with vitronectin, and Essential 8 (Invitrogen, registered trademark) was added as a cell proliferation medium at 4 mL/well, and cultured under 5% CO₂ at 37°C. The cell proliferation medium was changed daily until 10 days after culturing. On day 10 after culturing, the medium was replaced with Essential 6 (cell differentiation medium) containing no bFGF (basic fibroblast growth factor) and TGF-β1 (transforming growth factor-β1) (4 mL/well), up to 80 days after culturing, the cells were cultured under 5% CO₂ at 37°C, while the cell differentiation medium was changed twice a week. The amount of the indicator substance present in the culture supernatants of human ES cells and human iPS cells was measured over time by LC-MS. The analyzer used was LCMS-8050 (Shimadzu Corporation). The changes over time in the amount of each indicator substance after the start of culture is shown in FIGS. 6a to 6c: (A) ornithine, (B) citrulline, (C) glutathione, (D) cysteine, (E) pipecolic acid, (F). Putrescine, (G) proline, (H) 2-aminoadipic acid, (I) cytidine, (J) deoxycytidine, (K) adenosine, and (L) inosine.

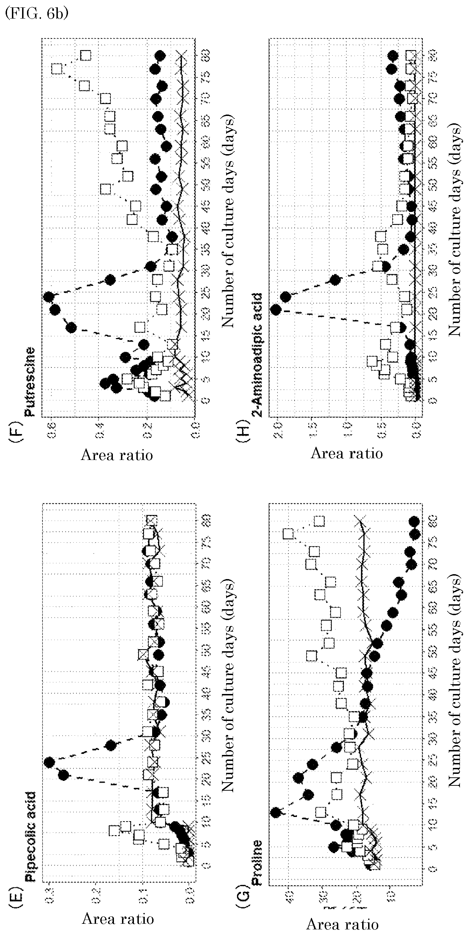

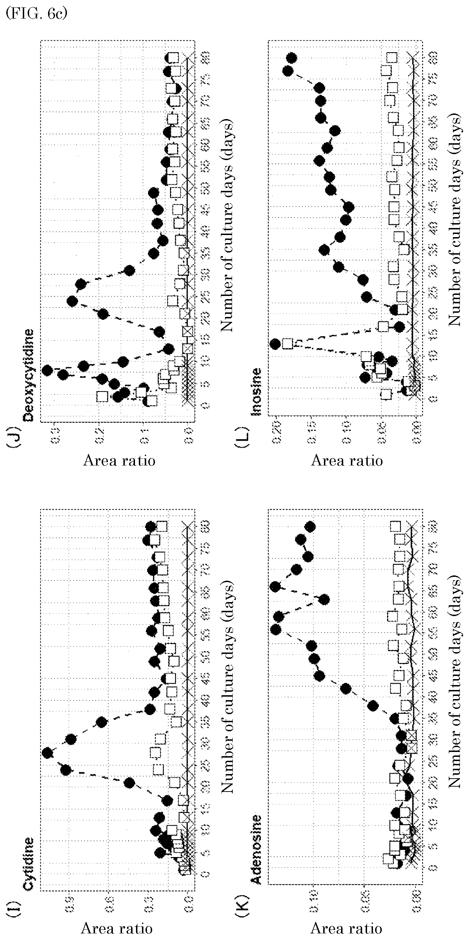

The cultured human ES cells was confirmed to differentiate into retinal pigment epithelial cells by the fact that the cell morphology became polygonal or paving stone-like, and pigmentation was observed around 42 days after the culture. On the other hand, no pigmentation was observed in the cultured human iPS cells, so it was judged that the cell did not differentiate. In comparison between human ES cells that finally differentiated into retinal pigment epithelial cells and human iPS cells that did not differentiate, it was found that the characteristics of the changes over time in amount of each indicator substance differed depending on the indicator substance. In human ES cells, the amount of glutathione (FIG. 6C) and cysteine (FIG. 6D) increased rapidly by 5 days after culture and then decreased. This gave a peak of the amount at a culture time of 1 to 10 days. In particular, glutathione was almost not produced in human iPS cells at a culture time of 1 to 10 days, which was significantly different from that of human ES cells. In human ES cells, the amount of deoxycytidine (FIG. 6J) rapidly increased by 8 days after culture and then decreased. This gave a peak of the amount at a culture time of 5 to 10 days. The amount of deoxycytidine also peaked during the culture time of 20-30 days. Ornithine (FIG. 6A) and citrulline (FIG. 6B) gave a unimodal peak in human ES cells at a culture time of 15 to 25 days, but no peak was observed in human iPS cells, indicating a significant difference. Pipecolic acid (FIG. 6E) showed a unimodal peak in human ES cells at culture times of 15-25 days, whereas it peaked in human iPS cells at culture times of 30-45 days. In addition, putrescine (FIG. 6F) and 2-aminoadipic acid (FIG. 6H) showed a unimodal peak in human ES cells at a culture time of 15 to 30 days, but the presence of putrescine in human iPS cells at the same time was low and did not show a clear peak. Cytidine (FIG. 6I) showed a distinct unimodal peak in human ES cells at culture times of 20-35 days. A peak-like increase in the amount of cytidine was also observed in human iPS cells, but the peak height was as small as about a quarter of that in human ES cells. The characteristic of the changes over time in the amount of the indicator substance is the changes before the differentiation of human ES cells into retinal pigment epithelial cells is confirmed by pigmentation. It is possible to determine in advance whether they differentiate or not, by measuring these indicator substances. Furthermore, by combining a plurality of these indicator substances, it is possible to improve the prediction accuracy of differentiation. The characteristics of the changes over time in the amount of each indicator substance described above in human ES cells differentiated into retinal pigment epithelial cells were not observed in human iPS cells that did not differentiate into retinal pigment epithelial cells.

Differentiation of cultured human ES cells into retinal pigment epithelial cells was confirmed at a culture time of around 42 days. After this culture time, an indicator substance showing a characteristic changes over time in the amount was found. Adenosine (FIG. 6K) and inosine (FIG. 6L) showed stable high values in human ES cells as compared with human iPS cells after the culture time of 42 days and subsequently thereafter when differentiation was confirmed. On the other hand, the amount of proline (FIG. 6G) showed a tendency to decrease in human ES cells after a culture time of 20 days and subsequently thereafter. In particular, after the culture time of 42 days and subsequently thereafter when differentiation was confirmed, the amount level was below the cell-free medium (blank). Since the characteristic changes over time of these indicator substances are not observed in human ES cells that did not differentiate into retinal pigment epithelial cells, it can be used to confirm that human ES cells have differentiated into retinal pigment epithelial cells.

## Claims

1. A method for evaluating the differentiation state of pluripotent stem cells in the process of inducing differentiation of pluripotent stem cells into retinal pigment epithelial cells, comprising
(1) a step of measuring the amount of the indicator substance present in the culture supernatant of pluripotent stem cells, and
(2) a step of evaluating the state of cell differentiation from pluripotent stem cells to retinal pigment epithelial cells based on changes in the amount of the indicator substance, where the indicator substances are ornithine and/or citrulline.

2. The method according to claim 1, wherein in step (2), the differentiation state of pluripotent stem cells is evaluated by analyzing the changes over time in the amount of the indicator substance present in the culture supernatant.

3. The method according to claim 2, wherein the period for analyzing the change over time is the period from day 0 to day 20, with the day when the medium for pluripotent stem cells is replaced from the medium for cell proliferation to the medium for cell differentiation set as day 0.

4. The method according to claim 2, wherein the period for analyzing the changes over time is the period from day 3 to day 12, with the day when the medium for pluripotent stem cells was replaced from the medium for cell proliferation to the medium for cell differentiation set as day 0.

5. The method according to any one of claims 2 to 4, wherein an assessment is made that cell differentiation are likely to progress from pluripotent stem cells to retinal pigment epithelial cells, when a determination is made that the changes over time are significant.

6. The method according to claim 1, wherein in step (1), the amount of ornithine and citrulline as the indicator substances is measured, and when the changes over time in the amounts of ornithine and citrulline are substantially the same, an assessment is made that cell differentiation from pluripotent stem cells to retinal pigment epithelial cells has progressed.

7. The method according to any one of claims 2 to 4, wherein in the step (2), the state of cell differentiation from pluripotent stem cells to retinal pigment epithelial cells is evaluated based on the threshold of the coefficient of variation regarding the amount of the indicator substance.

8. The method according to claim 7, wherein when the coefficient of variation threshold is 0.20 or more for the amount of ornithine and/or 0.30 or more for the amount of citrulline, an assessment is made that cell differentiation from pluripotent stem cells to retinal pigment epithelial cells progresses.

9. The method according to (1), further including step (3) of measuring the amount of the indicator substance present in the culture supernatant of the control cells whose state of cell differentiation is known, where
the state of cell differentiation from pluripotent stem cells to retinal pigment epithelial cells is evaluated, by comparing the amount of the indicator substance present in the culture supernatant of the pluripotent stem cells measured in step (1), with the amount of the indicator substance present in the culture supernatant of the control cells to be measured or had been measured in step (3).

10. The method according to claim 9, wherein the state of cell differentiation from pluripotent stem cells to retinal pigment epithelial cells is evaluated, based on whether the ratio or difference between the amount of the indicator substance present in the culture supernatant of the pluripotent stem cells and the amount of the indicator substance present in the culture supernatant of the control cells is equal to or greater than a predetermined threshold or less than the threshold.

11. The method according to claim 9, wherein the state of cell differentiation from pluripotent stem cells to retinal pigment epithelial cells is evaluated, by comparing the changes over time in the amount of the indicator substance present in the culture supernatant of the pluripotent stem cells and the changes over time in the amount of the indicator substance present of the control cells.

12. The method according to any one of claims 9 to 11, wherein the control cells are cells in which an undifferentiated state is maintained in the step of inducing differentiation of pluripotent stem cells into retinal pigment epithelial cells.

13. The method according to claim 1, wherein in the step of inducing differentiation of pluripotent stem cells into retinal pigment epithelial cells, the method further comprises step (4) of measuring in advance the amount of the indicator substance present in the culture supernatant of the control cells using cells in which cell differentiation has been confirmed as control cells,
and the state of cell differentiation is evaluated by comparing the amount of the indicator substance present in the culture supernatant of the pluripotent stem cells with the amount of the indicator substance present in the culture supernatant of the control cells.

14. The method according to claim 13, wherein the state of cell differentiation is evaluated based on a threshold value determined based on the amount of the indicator substance present in the culture supernatant of the control cells.

15. The method according to any one of claims 1 to 14, wherein the pluripotent stem cells are induced pluripotent stem cells (iPS cells) or embryonic stem cells (ES cells).

16. The method according to any one of claims 1 to 15, wherein the amount of the indicator substance is measured by mass spectrometry.

17. A method for producing retinal pigment epithelial cells from pluripotent stem cells using the method according to any one of claims 1 to 16.

18. A method for evaluating the differentiation state of ES cells in the step of inducing differentiation of embryonic stem cells (ES cells) into retinal pigment epithelial cells, comprising
(1) a step of measuring the amount of the indicator substance present in the culture supernatant of ES cells, and
(2) a step of evaluating the state of cell differentiation from ES cells to retinal pigment epithelial cells based on the change in the amount of the indicator substance, where
the indicator substance is at least one type selected from the group consisting of glutathione, ornithine, citrulline, cysteine, pipecolic acid, putrescine, proline, 2-aminoadipic acid, cytidine, deoxycytidine, adenosine, and inosine.

19. A method for evaluating the differentiation state of ES cells in the step of inducing differentiation of embryonic stem cells (ES cells) into retinal pigment epithelial cells, comprising
(1) a step of measuring the amount of the indicator substance present in the culture supernatant of ES cells, and
(2) a step of evaluating the state of cell differentiation from ES cells to retinal pigment epithelial cells based on the change in the amount of the indicator substance, where the indicator substance is at least one type selected from the group consisting of glutathione, ornithine, citrulline, cysteine, pipecolic acid, 2-aminoadipic acid, cytidine, and deoxycytidine.

20. A method for evaluating the differentiation state of ES cells in the step of inducing differentiation of embryonic stem cells (ES cells) into retinal pigment epithelial cells, comprising
(1) a step of measuring the amount of the indicator substance present in the culture supernatant of ES cells, and
(2) a step of evaluating the state of cell differentiation from ES cells to retinal pigment epithelial cells based on the change in the amount of the indicator substance, where the indicator substance is adenosine and/or inosine.

21. The method according to any one of claims 18 to 20, wherein in the step (2), the differentiation state of pluripotent stem cells is evaluated by analyzing the changes over time in the amount of the indicator substance present in the culture supernatant.

22. The method according to any one of claims 18 to 21, wherein the amount of the indicator substance is measured by mass spectrometry.

23. A method for producing retinal pigment epithelial cells from ES cells using the method according to any one of claims 18 to 22.
